# EUROPEAN PATENT SPECIFICATION

(11) **EP 2 210 611 B1**
(45) Date of publication and mention of the grant of the patent: **26.02.2014**
(21) Application number: 08836703.2
(22) Date of filing: 03.10.2008
(51) Int. Cl.: A61K 38/08, C07K 14/33, A61P 25/28

(54) **USE OF THE SEQUENCE ENCODING THE CARBOXYL-TERMINAL DOMAIN OF THE HEAVY CHAIN OF TETANUS TOXIN AS A DRUG**
VERWENDUNG DER SEQUENZKODIERUNG DES CARBOXYL-TERMINALEN BEREICHS DER SCHWEREN KETTE DES TETANUSTOXINS ALS WIRKSTOFF
UTILISATION DE LA SÉQUENCE CODANTE DU DOMAINE CARBOXY-TERMINAL DE LA CHAÎNE LOURDE DE LA TOXINE TÉTANIQUE COMME MÉDICAMENT

(30) Priority: 05.10.2007 ES 200702621
(43) Date of publication of application: 28.07.2010
(60) Divisional application: 13181260.4
(73) Proprietor: Universidad De Zaragoza, 50009 Zaragoza (ES); Universitat Autònoma De Barcelona, 08193 Barcelona (ES)
(72) Inventor: MORENO IGOA, María, E-50009 Zaragoza (ES); CALVO ROYO, Ana Cristina, E-50009 Zaragoza (ES); MUÑOZ GONZALVO, Mª Jesús, E-50009 Zaragoza (ES); ZARAGOZA FERNÁNDEZ, Mª Pilar, E-50009 Zaragoza (ES); AGUILERA AVILA, José, E-08193 Bellaterra (ES); OSTAS PINZOLAS, Rosario, E-50009 Zaragoza (ES)
(74) Representative: Chapman, Paul William
(86) International application number: PCT/ES2008/070186
(87) International publication number: WO 2009/043963

(56) References cited:
- EP-A2- 0 057 140
- WO-A1-2007/082973
- WO-A2-99/09057
- WO-A2-2004/021992
- WO-A2-2005/025592
- WO-A2-2008/094583
- LARSEN K E ET AL: "A glial cell line-derived neurotrophic factor (GDNF):tetanus toxin fragment C protein conjugate improves delivery of GDNF to spinal cord motor neurons in mice" BRAIN RESEARCH, ELSEVIER, AMSTERDAM, NL LNKD- DOI:10.1016/J.BRAINRES.2006.08.079, vol. 1120, no. 1, 20 November 2006 (2006-11-20), pages 1-12, XP025101816 ISSN: 0006-8993 [retrieved on 2006-11-20]
- BORDET T ET AL: "NEURONAL TARGETING OF CARDIOTROPHIN-1 BY COUPLING WITH TETANUS TOXIN C FRAGMENT" MOLECULAR AND CELLULAR NEUROSCIENCES, SAN DIEGO, US LNKD- DOI:10.1006/MCNE.2001.0979, vol. 17, no. 5, 1 May 2001 (2001-05-01), pages 842-854, XP001206036 ISSN: 1044-7431
- DATABASE BIOSIS [Online] BIOSCIENCES INFORMATION SERVICE, PHILADELPHIA, PA, US; 2003, CHIAN R ET AL: "Insulin - like growth factor - 1:tetanus toxin fragment C fusion protein for improved delivery of IGF - 1 to the CNS." XP002607642 Database accession no. PREV200400199532 & SOCIETY FOR NEUROSCIENCE ABSTRACT VIEWER AND ITINERARY PLANNER, vol. 2003, 2003, pages Abstract No. 413.14 URL-http://sf, 33RD ANNUAL MEETING OF THE SOCIETY OF NEUROSCIENCE; NEW ORLEANS, LA, USA; NOVEMBER 08-12, 2003
- CHAIB OUKADOUR I ET AL: "The C-terminal domain of the heavy chain of tetanus toxin rescues cerebellar granule neurons from apoptotic death: involvement of phosphatidilinositol 3-kinase and mitogen-activated protein kinase pathways" JOURNAL OF NEUROCHEMISTRY, WILEY INTERSCIENCE, NEW YORK, NY, US LNKD- DOI:10.1111/J.1471-4159.2004.02586.X, vol. 90, no. 5, 1 May 2004 (2004-05-01), pages 1227-1236, XP003015965 ISSN: 0022-3042
- BORDET T ET AL: "Protective effects of cardiotrophin-1 adenoviral gene transfer on neuromuscular degeneration in transgenic ALS mice" HUMAN MOLECULAR GENETICS, OXFORD UNIVERSITY PRESS, SURREY LNKD- DOI:10.1093/HMG/10.18.1925, vol. 10, no. 18, 1 September 2001 (2001-09-01), pages 1925-1933, XP002347773 ISSN: 0964-6906
- LONGSTRETH W T ET AL: "Hypothesis: A motor neuron toxin produced by a clostridial species residing in gut causes ALS" MEDICAL HYPOTHESES, EDEN PRESS, PENRITH, US LNKD- DOI:10.1016/J.MEHY.2004.07.041, vol. 64, no. 6, 1 January 2005 (2005-01-01), pages 1153-1156, XP004846343 ISSN: 0306-9877
- MARÃ ÂA MORENO-IGOA ET AL: "Fragment C of tetanus toxin, more than a carrier. Novel perspectives in non-viral ALS gene therapy" JOURNAL OF MOLECULAR MEDICINE, SPRINGER, BERLIN, DE, vol. 88, no. 3, 18 November 2009 (2009-11-18), pages 297-308, XP019791482 ISSN: 1432-1440
- LARSEN, K.E. ET AL.: 'A glial cell line-derived neurotrophic factor (GDNF):tetanus toxin fragment C protein conjugate improves delivery of GDNF to spinal cord motor neurons in mice' BRAIN RESEARCH vol. 1120, no. 1, 2006, pages 1 - 12, XP025101816
- BORDET, T. ET AL.: 'Neuronal targeting of cardiotrophin-1 by coupling with tetanus toxin C fragment' MOLECULAR AND CELLULAR NEUROSCIENCE vol. 17, no. 5, 2001, pages 842 - 854, XP001206036
- FRANCIS, J.W. ET AL.: 'CuZn Superoxide Dismutase (SOD-1):Tetanus Toxin Fragment C Hybrid Protein for Targeted Delivery of SOD-1 to Neuronal Cells' THE JOURNAL OF BIOLGICAL CHEMISTRY vol. 270, no. 25, 1995, pages 15434 - 15442, XP008132949

## Description

This invention concerns the use of the sequence encoding of the carboxyl-terminal domain of the heavy chain of the tetanus toxin as a drug, preferably in the treatment of Amyotrophic Lateral Sclerosis (ALS), as well as the polypeptide encoded by said sequence for the treatment of the aforementioned disease.

### BACKGROUND OF THE INVENTION

Amyotrophic Lateral Sclerosis (Lou Gehrig's or Charcot's Disease) is a progressive, incurable, and fatal disease that progresses to the degeneration of the motor neurons at the medullar, bulbar, and motor cortex level. In the case of Spain, the illness presents an incidence of 2/100,000 and a prevalence of 1/10,000, which indicates that approximately 40,000 Spaniards will develop the disease at some time during their lifetime (source: Spanish Amyotrophic Lateral Sclerosis Association -ADELA-).

Despite having been a recognized disease for a long time, it is still not precisely known what causes it and, even though there are genetic forms of this disease, cases are also known which appear to have no hereditary origin. Therefore, it is estimated that 10% of cases are of genetic origin, the familiar forms, of which 15-20% correspond to mutations in the Superoxide Dismutase enzyme (SOD-1.) and mutations in this enzyme have even been observed in sporadic forms of the illness (Brown, R.H. Jr. (1997). Arch. Neurol. 54(10) 1246-1250). Mutations in the NFH, the gene that encodes the neurofilament heavy chain, have also been found in an exceptional form in some sufferers of Amyotrophic Lateral Sclerosis.

Consequently, research into genetic inheritance of this disease is of great interest.

In recent years, the creation of animal models of the illness has become one of the most relevant tools in experimental treatment studies, which have helped clear up some questions about its causes, although the causes are still greatly misunderstood. Neither knockout mice for the SOD-1 enzyme, nor transgenic animals for different mutations in the human SOD-1 enzyme have been able to reproduce a clinical pattern similar to the disease in humans. The animal that most closely approximates the development of the illness is a transgenic that presents several copies of mutant superoxide dismutase in its 93 position, is the so called SOD1G93A (Tu, P.H., et al. (1996.) Proc. Nati. Acad. Sci. USA. 93(7): 3155-3160.) which is supplied by the Jackson Laboratory.

In spite of numerous studies carried out in order to understand its cause as well as its mechanism, at the moment, no classic effective treatments exist. Currently, three lines of research are under development based on the application of glutamate antagonists, neurotrophic factors and antioxidants, even though to date none of these have lead to an effective treatment.

For several years now the capacity of neurotrophic factors has been known to rescue motor neurons from degeneration. Great interest has resulted from experiences with genetic therapies performed in animal models, using adenoviral vectors that express various neurotrophic factors (GDNF, CNTF, NI-4, IGF-1), which have offered hopeful results. Nevertheless, adenoviral injections have presented the disadvantage of having to be applied to neonatal animals due to the great immune response that they create. Therefore, although the results have been hopeful, developing new vectors which are less immunogenic is imperative in order to make an effective treatment for ALS possible.

In regard to the clinical tests carried out to date, at the beginning of 1996 by Dr. Schuelp, satisfactory results were not obtained (http://www.wiley.co.uk/genetherapy), with possible causes of this failure: the nature of the neurotrophic factor used in the testing (CNTF) and/or its lack of accessibility to the Central Nervous System. In 1999, Dr. Axel Kahn's group proved that the administration route for said neurotrophic factor in animal models is in fact an important factor in its therapeutic effect (Haase et al. (1999) Ann. Neurol. 45(3) 296-304). This lack of specificity has also been proposed as the probable cause of the failure in the administration of BDNF in humans in subcutaneous form.

Moreover, neurotrophic factors administered in a systemic way present problems with toxicity when acting upon other tissues. In spite of all these disadvantages, the therapeutic possibilities of neurotrophic factors continue to be researched due to their promising results in the preclinical phase. Specifically, the latest clinical trial taking place at the Rochester Medical Center (Minnesota) is once again based on the administration of a neurotrophic factor, IGF-1.

WO2004/021992 describes the use of the carboxyl-terminal domain of the non-toxic heavy chain of the tetanus toxin (HcTeTx) to target proteins to the CNS.

Larsen et al, Brain Research, 1120 No.1, (2006) p1-12, describes the use of the tetanus toxin C-terminal fragment as a delivery vehicle for delivering exogenous proteins to CNS motor neurons.

Bordet et al, Molecular and Cellular Neurosciences, San Diego, US, Vol 17, No.5, (2001) p842-854 describes a fusion protein of insulin-like growth factor-1 and tetanus toxin fragment C, for improved delivery of IGF-1 to the CNS.

Chian et al, Society for Neuroscience, 33d Annual Meeting, New Orleans, USA, (2003), Abstract No.413, discloses the use of the tetanus C-terminal fragment as a delivery vehicle for delivery of exogenous proteins to CNS motor neurons.

### DESCRIPTION OF THE INVENTION

The authors of this invention have described that the non-toxic carboxyl terminal domain of the heavy chain of the tetanus toxin (HcTeTx), (that to date has only been used in the treatment of ALS as a vehicle for various neurotrophic factors, as well as the SOD-1 enzyme, through the creation of fusion proteins) is by itself capable of prolonging the survival of animal models of the disease.

Thus, a first aspect of the invention provides use of an isolated polynucleotide consisting of the encoding sequence of the carboxy-terminal domain of the heavy subunit of the tetanus toxin (HcTeTx), or a fragment of the encoding sequence, or a polynucleotide variant thereof, as a therapeutic agent in the manufacture of a medicament for the treatment of the symptoms of Amyotrophic Lateral Sclerosis (ALS), wherein
(a) the polypeptide encoded by the fragment or the polynucleotide variant maintains a therapeutic effect on ALS;
(b) the sequence of the polypeptide encoded by said polynucleotide variant is at least 85% identical to the sequence of SEQ ID NO:2 or SEQ ID NO:5;
(c) the polypeptide encoded by the isolated polynucleotide does not form part of a fusion protein; and
(d) the HcTeTx, HcTeTx fragment, or polypeptide variant encoded by the isolated polynucleotide is therapeutically effective by itself.

In one preferred embodiment of the invention, the encoding sequence of HcTeTx encompasses the encoding triplet of the amino acid V (Valine) of the amino terminal end of HcTeTx to the triplet that encodes the amino acid D (Aspartate), preferably from the amino acid V (854) to D (1315) of the sequence with access number (NCBI.: P04958). In an even more preferred embodiment of this aspect of the invention, the encoding sequence of HcTeTx is SEQ ID NO: 1 and the HcTeTx fragment is SEQ ID NO: 5. Hereinafter, this polynucleotide shall be called the "*polynucleotide of the invention*".

In another preferred embodiment, the polynucleotide of the invention may be a mutation (deletion, insertion, inversion, point mutation, etc.) where said mutations do not affect its capacity to act as a drug, specifically for the treatment of ALS. Maintenance of the therapeutic effect of the mutated polynucleotide of the invention may be tested through the reproduction of any of the examples I and II. Throughout the description these mutated polynucleotides shall be also considered as allelic variations.

In an also preferred embodiment, the polynucleotide of the invention may also include promoting, termination, silencing sequences; sequences that facilitate their integration into chromosomes or any type of organizational structure of genetic material, etc.

In a second aspect the invention provides use of an isolated polypeptide consisting of the carboxy-terminal domain of the heavy subunit of the tetanus toxin (HcTeTx), a HcTeTx fragment, or a polypeptide variant, as a therapeutic agent, for the manufacture of a medicament for the treatment of the symptoms of Amyotrophic Lateral Sclerosis (ALS), wherein:
(a) the HcTeTx fragment, or the polypeptide variant maintain a therapeutic effect on ALS;
(b) the sequence of the polypeptide variant is at least 85% identical to the sequence of SEQ ID NO:2 or SEQ ID NO:5;
(c) the HcTeTx, HcTeTx fragment, or polypeptide variant do not form part of a fusion protein; and,
(e) the HcTeTx, HcTeTx fragment, or polypeptide variant is therapeutically effective by itself.

The invention also refers to the use of a vector that includes the polynucleotide of the invention for the creation of a drug, preferably for the treatment of ALS, where said vector is selected from the group including (with no type of limitation), plasmids, phages, cosmids, phagemids, artificial yeast chromosomes (YAC), artificial bacterial chromosomes (BAC), artificial human chromosomes (HAC), viral vectors, such as adenovirus, retroviruses, or any other type of DNA or RNA molecule capable of replicating itself inside a prokaryote or eukaryote cell. Hereinafter this vector shall be called "vector of the invention".

The invention also refers to the use of a transgenic cell for the creation of a drug, preferably the treatment of ALS, in which said cell includes the polynucleotide of the invention or the vector of the invention.

The invention relates to the use of an isolated polynucleotide that includes the encoding sequence of isolated HcTeTx, its allelic variations, or its function fragments of same for the creation of a drug for the treatment of ALS. In a preferred embodiment of the invention, the HcTeTx sequence encompasses from the amino acid V(854) to D(1315) of the sequence with the access number (NCBL: P04958). In an even more preferred embodiment of this aspect of the invention, the sequence of HcTeTx is SEQ ID NO: 2 and the HcTeTx fragment is SEQ ID NO: 6. Hereinafter, this polynucleotide shall be called the "*polynucleotide of the invention*"*.*

In another preferred embodiment, the polypeptide of the invention is mutated (deletion, insertion, inversion, point substitutions of amino acids, etc.), even though said mutations do not affect their capacity to act as a drug for the treatment of ALS. Maintenance of the therapeutic effect of the mutated polynucleotide of the invention may be tested through the reproduction of the examples 1 and 2.

For administration of the drug or the pharmaceutical compound, the polynucleotide, the vectors, the transgenic cells, or the polypeptide of the invention will be formulated in a pharmaceutical form suitable for its administration using the chosen route of administration. For this, said pharmaceutical compound shall include the vehicles and excipients which are pharmaceutically acceptable and necessary to create the selected pharmaceutical form of administration. Information on excipients or vehicles that may be used in the creation of said pharmaceutical compounds, as well as on pharmaceutical forms of administration of the active ingredients, in general, can be found in the book, "Treatise of the Galenic Pharmacy", by C. Faulí i Trillo, 1st Edition, 1993, Luzan 5, S.A. de Ediciones.

Said pharmaceutical composition includes, at least, any of the elements from the group including: the polynucleotide, vectors, transgenic cells, or the polypeptide of the invention should be found in an therapeutically effective amount. In the sense used in this description, the expression "therapeutically effective amount" refers to the amount of the element selected calculated to produce the effect desired and, in general, will be determined by the characteristics of the polypeptide element itself, among other causes, and the therapeutic effect to be obtained, the characteristics of the individual to be treated, the severity of the illness suffered by said individual, etc. Hereinafter this pharmaceutical compound shall be said "*pharmaceutical compound or drug of the invention*".

The pharmaceutical compound of the invention may be administered by any suitable route of administration, for example, by oral, parenteral, nasal (mucosa) route, etc., typically, by parenteral route, advantageously, through its intramuscular or subcutaneous administration. Also, said pharmaceutical compound may present any form of presentation suitable for its administration, for example, in solid form (tablets, capsules, granules, etc.), liquid (solutions, suspensions, emulsions, etc.), etc., for its administration via the route of administration selected. In one embodiment preferred, said pharmaceutical compound is formulated in a pharmaceutical form for an appropriate unit dosage.

In a preferred embodiment, the pharmaceutical compound may be in a pharmaceutical form to be administered by oral route, either in solid form, preferably liquid, more preferably ready for its administration via intramuscular route. Illustrative examples of pharmaceutical forms of administration by oral route including tablets, capsules, granules, solutions, suspensions, etc., and may contain conventional excipients, such as agglutinates, diluents, disintegrants, lubricants, humectants, etc., and may be prepared by conventional methods. In another preferred embodiment, the pharmaceutical compounds may also be adapted for parenteral administration, in form of, for example, solutions, suspensions, or lyophilized, sterile products, in suitable dosage form; in this case, said pharmaceutical compounds will include suitable excipients, such as buffers, surfactants, etc. In any case, the excipients shall be selected in the context of the pharmaceutical form of administration selected. A review of the various pharmaceutical forms of administration of drugs and their preparation may be found in the book, "Treatise of the Galenic Pharmacy", by C. Faulí i Trillo, 10th Edition, 1993, 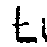 uzan 5, S.A. de Ediciones, cited above. Also, the pharmaceutical compound may include other polypeptides, polynucleotides, vectors, or cells that offer a greater efficiency and to the compound.

### Definitions:

The term "polynucleotide", as used in this document, refers to a polymeric form of nucleotides of any length, and may be deoxyribonucleotides or ribonucleotides. This term refers exclusively to the primary structure of the molecule. Thus, this term includes DNA bi- and mono-catenary, as well as RNA bi- and mono-catenary.

The term "isolated" throughout the description when it is used in association with HcTeTx or its encoding sequence, not only refers to the fact that these are found isolated from the human body, but also that they do not form part of the fusion proteins or enzymes that will carry out a therapeutic function.

The expression "functional fragment of HcTeTx, allelic variants of same, or the sequences that encode them" is referring throughout the description to a peptide or a polynucleotide that includes a portion of HcTeTx, its allelic variants or its encoding sequences, that maintains their capacity to act as a drug, more specifically for the treatment of ALS, where the maintenance of their therapeutic capacity may be tested through the reproduction of the examples 1 - 3.

The term "allelic variant" throughout the description refers to a polypeptide that is very homologous and functionally equivalent to the C-terminal domain of the heavy chain of the tetanus toxin. As is used here, a peptide is "very homologous" to said domain when its sequence of amino acids have a grade of identity regarding the sequence of amino acids of said domain of, at least, 60%, 70%, 85% and, more preferably of, at least, 95%. Preferably the amino acid sequence of said domain is SEQ ID NO: 2. This term is also referred to in the description as a polynucleotide capable of encoding a very homologous polypeptide that is functionally equivalent to HcTeTx. In this way, the polynucleotide may have a homology of at least 40%, 50%, 60%, 70%, 85% or 95% with the encoding polynucleotide of HcTeTx, the nucleotide sequence of which is preferably the SEQ ID NO: 1.

The expression "functionally equivalent" as is used throughout the description, means that the polypeptide or the polypeptide [SIC] maintains its capacity to act as a drug, more specifically for the treatment of ALS, when maintenance of its therapeutic capacity may be tested through the reproduction of example I or II.

For experts in the material, other objects, advantages, and characteristics of the invention are broken down in part of the description and in part of the practice of the invention. The following examples provide a mode of illustration, and they should not be considered as limiting this invention.

### BRIEF DESCRIPTION OF THE FIGURES

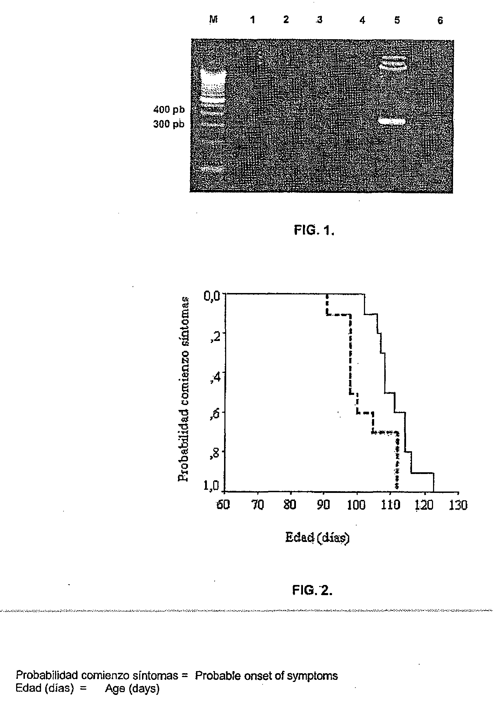

**Figure 1**. PCR amplification for the detection of the HcTeTx expression. Ten days after the intramuscular injection of the plasmid pCMV-HcTeTx (n=2, lanes 1 and 2) and of the empty plasmid pCMV (n=2, lanes 3 and 4) RNA was extracted from the muscle and retro-transcription was carried out. The cDNA obtained was amplified by PCR for the gene HcTeTx. Lane 5 shows the positive control (plasmid pCMV-HcTeTx) and in lane 6 the target of the reaction is loaded. In lane M the size marker 100 pb is found.
**Figure 2**. Effects of the treatment with naked DNA encoding HcTeTx at the onset of symptoms in model mice for the ALS SOD1 G93A disease. The manifestation of the symptoms was significantly delayed in the group treated with HcTeTx (n=10) in comparison with the control group (n=10). The accumulated probabilities were calculated using the Kaplan-Meier Survival Analysis (SPSS 13.0).

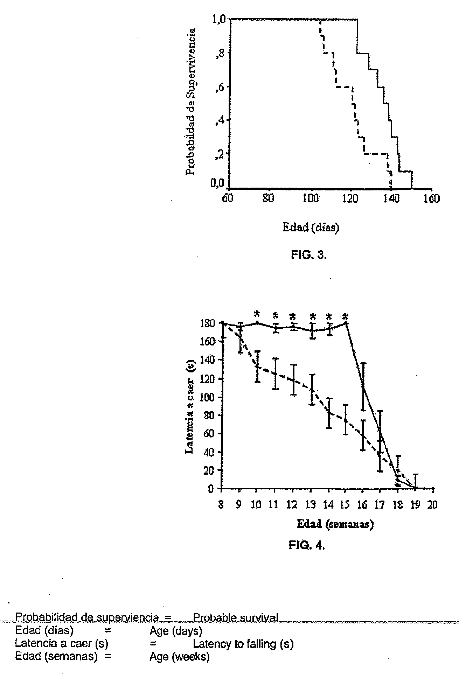

**Figure 3**. Effects of the treatment with naked DNA encoding HcTeTx upon survival in model mice for the ALS SOD1G93A disease. Survival increased significantly in the group treated with HcTeTx (n=10) in comparison with the control group (n=10). The accumulated probabilities were calculated using the Kaplan-Meier Survival Analysis (SPSS 13.0).
**Figure 4**. Effects of the treatment with naked DNA encoding HcTeTx. The motor activity was determined through the rotarod at a constant velocity of 14 rpm, with a maximum time of development of 180s. Greater motor activity was observed in the group treated with HcTeTx (n=10) in comparison with the control group (n=10).

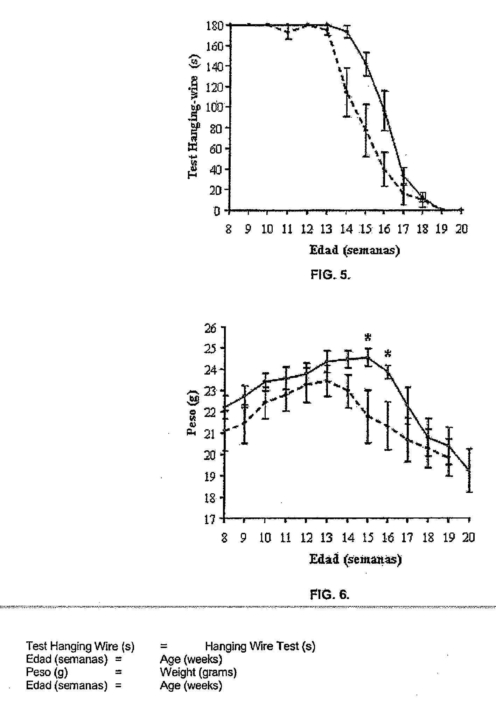

**Figure 5****.** Effect of the intramuscular injection of naked DNA encoding HcTeTx in SOD1-G93A mice. The motor force and function in the mice were tested using the hanging wire test. 10 mice were used in each group (n=10).
**Figure 6**. Effect of the intramuscular injection of naked DNA encoding HcTeTx in SOD1-G93A mice. Measurements of the weight of the transgenic mice treated with HcTeTx. 10 mice were used in each group (n=10).

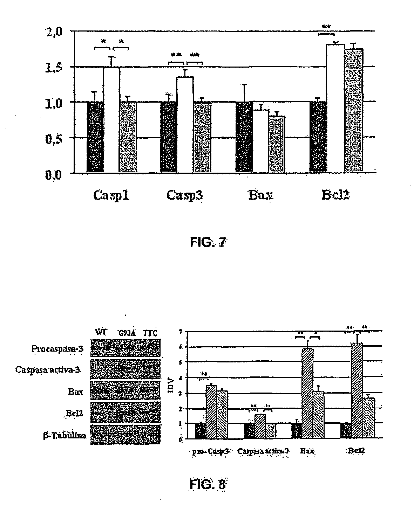

**Figure 7**. Analysis of the gene expression involved in the apoptosis signaling pathway in the spinal cord of the symptomatic SOD1 G93A mice that were 110 days old. Representation of the average messenger RNA values in the Caspl, Casp3, Bax and Bcl2 genes and the control (target) and the mice treated with HcTeTx (gray). The previous mice groups were compared with wild-type mice (black) (n=5 mice per group).
**Figure 8**. Analysis of the proteins involved in the apoptosis signaling pathway in the spinal cord of the symptomatic SOD1 G93A mice that were 110 days old. Western-Blot analysis of the pro-Casp3, active Casp3, Bax and Bcl2 in proteins used in the spinal cord of mice treated with HcTeTx (gray lines) and control mice (black lines) compared with wild-type mice (black) (n=5 mice per group).

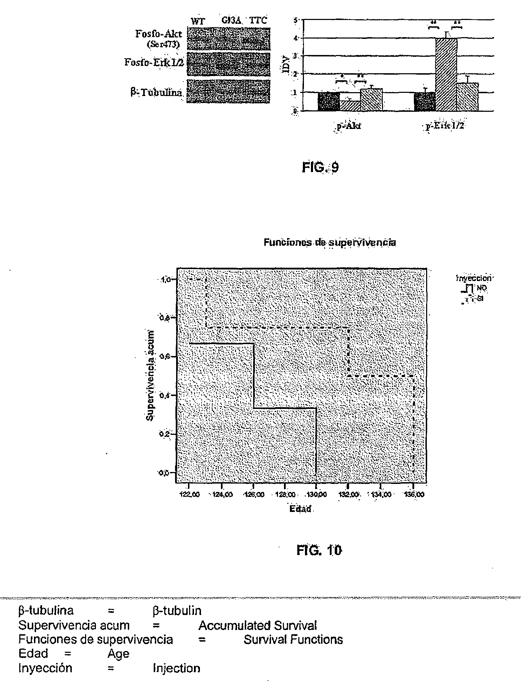

**Figure 9****.** Western-Blot analysis of the phosphorilation of the Akt and Erk 1/2 proteins. Samples of 5 mice per group were analyzed. IDV *(Intensity Density Value)*. The amounts analyzed by Western-Blot are shown as the ratio relative to p-tubulin regarding the values of the wild-type mice. (**P*<0.05, ***P*<0.01; error bars indicate the SEM). The bars represent the same groups that have been described in the previous caption.
**Figure 10**. Effects of the intraperitoneal treatment with the polypeptide that contains the C-terminal domain of the heavy chain of the tetanus toxin (HcTeTx) on the survival in model mice for the ALS SOD1 G93A Survival increased significantly in the group treated with HcTeTx (n=3) in comparison with the control group (n=3, continuous line). The accumulated probabilities were calculated using the Kaplan-Meier Survival Analysis (SPSS 13.0).

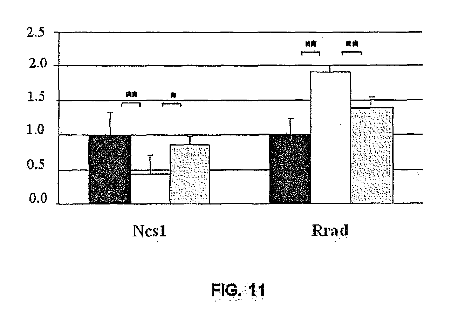

**Figure 11**. Intramuscular treatment of mice injected with HcTeTx affects the expression of genes related to homeostasis of calcium in the spinal cord of SOD1G93A transgenic mice. The levels of expression in genes Ncs1 and Rrad in transgenic mice treated with HcTeTx (gray) or the empty plasmid (white) were determined. Changes in the levels of messenger RNA in the prior groups of mice were compared with the wild-type mice (black). (**P*<0.05, ***P*<0.01; error bars indicate the SEM; n=5 mice per group).

### DETAILED DISCLOSURE OF THE EMBODIMENTS

Below the invention is illustrated through tests carried out by the inventors, which demonstrate the efficiency of HcTeTx, as well as their encoding sequence, for its use as a drug, and more preferably for the treatment of ALS.

### EXAMPLE 1

### The administration of HcTeTx via intramuscular injection of naked DNA slows the onset of symptoms and prolongs the survival in SOD1G93A mice.

The generation of transgenic animals that overexpress the gene for human superoxide dismutase-1 (SOD-1) with different mutations has provided animal models for the study of the disease ALS. These animals present clinical and pathological characteristics such as those suffering from ALS. One of the models most studied and characterized is the SOD1 G93A transgenic mice, that present a mutation substituting glycine amino acid with alanine in position 93 on the gene for the SOD-1 enzyme. This model animal has been successfully tested using various therapeutic compounds. However this has not translated into an effective therapy in human clinical trials, either due to an inadequate route of administration and/or due to the limited bioavailability of therapeutic molecules to reach the target cells. Some gene therapy strategies include the use of the adeno-associated virus, (AAV), which is transported in a retrograde manner to motor neurons via intramuscular injection. However the possibility of using viral vectors still exists, which may cause additional damage to sufferers treated. The use of naked DNA is a more secure and appropriate alternative strategy for delivering a specific gene therapy to patients.

### Materials and methods

### 1.1 Naked DNA encoding HcTeTx

The gene encoding HcTeTx (C-terminal domain of the heavy chain of the tetanus toxin -SEQ ID NO: 2 of 462 amino acids-) were cloned in the eukaryote plasmid expression pcDNA3.1 (invitrogen), under control of the cytomegalovirus (CMV) promoter. The vectors were produced in the chemically competent bacteria *Escherichia coli* (DH5a) and were purified using the Sigma-Aldrich maxiprep GenElute kit.

### 1.2 Transgenic mice

Transgenic mice that overexpress human SOD1 with the G93A mutation (B6SJL-TgN[SOD1-G93A]1Gur) were obtained from the Jackson Laboratory (Bar Harbor, ME). Hemizygote mutants were used in all of the experiments (a mutant male mated to a non-transgenic female). The transgenic mice were identified by PCR amplification of the DNA extracted from the tail, as described in Gurney et al. (Gurney et al., 1994. Motor neuron degeneration in mice that express a human Cu, Zn superoxide dismutase mutation. Science, 264 (5166): 1772-5). The animals were preserved in a Mixed Research Unit at the University of Zaragoza. They were given water and food ad libitum. All the experiments performed on and the care given to the animals were developed in accordance with the standards of the University of Zaragoza and the international guide for the use of laboratory animals.

### 1.3 Naked DNA intramuscular injection and extraction of the muscle

At the age of 8 weeks the SOD1G93A transgenic mice were injected intramuscularly with 300 µg of pCMV-HcTeTx in the cuadriceps muscle (two injections of 50 µg per muscle) and in the triceps muscle (one single injection of 50 µg per muscle). The control group of mice was injected with the same amounts of an empty plasmid. Ten days after the intramuscular plasmid injections, the inoculated muscles were extracted that were pre-frozen in liquid nitrogen and later stored at -70 °C.

### 1.4 Extraction of RNA, synthesis of cDNA and PCR amplification

The tissues were frozen in liquid nitrogen and were ground to a powder thereafter in a cold mortar. The total muscle RNA was extracted in accordance with the TRlzol Reagent (Invitrogen) Protocol. For cDNA synthesis, the SuperScriptTM First-Strand Synthesis System (Invitrogen) kit was used, beginning with 1 µg of RNA in a final volume of 20µL. The PCR reactions were carried out in a final volume of 20µL, with 150nM in each initiator, 150µM of dNTPs, 2mM de MgCl₂, 1 X buffer, 0.2U Taq pol and 2µL per cDNA reaction diluted 10 times for the amplification of a HcTeTx gene fragment. All the PCR reactions were carried out in en GeneAmp® Thermal Cycler 2720 (Applied Biosystems, Foster City, CA, USA). The thermal cycle parameters were as follows: incubation at 94°C for 3 min and 35 cycles at 94°C for 30s, 61 °C for 30s and 72°C for 30s. The presence of the amplification of the HcTeTx gene was observed in an agar gel tinted with 2% Ethidium Bromide. The direct and reverse initiator sequence used were SEQ ID NO: 3 and SEQ ID NO: 4, respectively. The amplicon size was 355 pb.

### 1.5 Rotarod text, grid and survival test_{.}

The grid test was used to determine the muscular strength and the onset of the ALS symptoms. The animals performed this test once a week from the age of 8 weeks. Each mouse was placed on a grid that was used as a lid for conventional cages. The grid was then turned 180° and maintained at a distance of approximately 60 cm, from a soft surface to avoid any injuries. The latency of each mouse falling was timed. Each of the mice was given up to three tries to hold on to the inverted grid for a maximum of 180s and the longest time was recorded.

The rotarod test was used to evaluate motor coordination, strength, and balance. The animals were placed on the rotating rod on the equipment (ROTA-ROD/RS, LE8200, LSI-LETICA Scientific Instruments). The time was recorded during which an animal was able to remain on said rod at a constant velocity of 14 rpm. Each mouse had three chances and the longest time the animals were able to remain without falling from the rod was recorded, with an arbitrary time limit of 180s.

The final point in the life of the mice was considered to be when the animals were placed in a supine position and were not able to turn themselves over.

### Results

### 2.1 Detection of the plasmid expression in the muscle

Initially the capacity of the constructed pCMV-HcTeTx to express the encoding gene in the muscle cells of the SOD1 G93A transgenic mice was confirmed. Due to there being no existence of the endogenous expression of the HcTeTx gene in these mice, the PCR amplification of a fragment of this gene was applied to the muscles by injection in order to detect the mRNA expression of said molecule. As shown in Figure 1., the expression of the gene HcTeTx was not observed in the control group injected with empty plasmid. However PCR revealed the presence of the amplification of the HcTeTx gene in the muscle inoculated with the encoding vector for same, indication that the vector successfully reached the muscle cells and that the process of transcription of said gene took place.

### 2.2 HcTeTx delays the manifestation of symptoms, improves motor capacity and prolongs survival of the SOD1 G93A transgenic mice

Intramuscular treatment with naked DNA encoding HcTeTx produced a delay in the onset of symptoms, improved motor activity and postponed the final point of the disease in the ALS model mouse, which contains the G93A mutation and the human SOD1 gene. The manifestation of the symptoms was recorded as well as the first day on which the mice could not remain on the inverted grid for three minutes. The onset of the symptoms was significantly reduced in approximately 8 days in the group of animals injected with HcTeTx, as compared to the control group (Figure 2, and Table 1.). As can be seen in Figure 3, and in Table 1, maximum survival was detected in the mice from the group treated with HcTeTx, that achieved and average of 136 days; 16 days longer than the control group. Between weeks 12 and 13 a significant decrease was seen in the development of activity on the rotarod in the control group, while these deficiencies were not observed in the group of treated animals until week 16 (Figure 4).

**Table 1. Table where the data concerning manifestation of symptoms and survival are gathered, in the control group as well as the group treated with HcTeTx, as well as the P Value (Log Rank, Mantel-Cox).**

| | **Control** (n=10) | **HcTeTx** (n=10) | ***P Value*** |
|---|---|---|---|
| **Onset of symptoms (days)** | 102.4 ± 2.4 | 110.9 ± 2.0 | 0.0295 |
| **Mortality (days)** | 120.5 ± 3.9 | 136.0 ± 3. | 0.0093 |
| **Difference in onset-mortality (days)** | 18.1 | 25.1 | |

The treatment was also evaluated in mice beginning at the age of 8 weeks using the "hanging wire" test (Figure 5). At 14 weeks of age, the SOD1G93A mice showed the first signs of weakness, while the group of mice treated with HcTeTx were show to be stronger between weeks 14-16. Also, the control group mice began to lose weight at 14 weeks of age which was associated with the disease. However, treatment with HcTeTx significantly reversed the weight loss, showing a maximum weight at 15 weeks (Figure 6).

### EXAMPLE 2

### Inhibition of apoptosis in the spinal cord of the SOD1G93A mice treated using an intramuscular naked DNA encoding HcTeTx injection

### Materials and methods

### 1.1 Naked DNA encoding for HcTeTx

The gene encoding HcTeTx (C-terminal domain of the heavy chain of the tetanus toxin -SEQ ID NO: 1) were cloned in the eukaryote plasmid expression pcDNA3.1 (Invitrogen), under control of the cytomegalovirus (CMV) promoter. The vectors were produced in the chemically competent bacteria *Escherichia coli* (DH5a) and were purified using the Sigma-Aldrich Genelute maxiprep kit.

### 1.2 Transgenic mice

Transgenic mice that overexpress human SOD1 with the G93A mutation (B6SJL-TgN[SOD1-G93A]1Gur) were obtained from the Jackson Laboratory (Bar Harbor, ME). Hemizygote mutants were used in all of the experiments (a mutant male mated to a non-transgenic female). The transgenic mice were identified by PCR amplification of the DNA extracted from the tail, as described in Gurney et al. (Gurney et al., 1994. Motor neuron degeneration in mice that express a human Cu, Zn superoxide dismutase mutation. Science, 264 (5166): 1772-5). The animals were preserved in a Mixed Research Unit at the University of Zaragoza. They were given water and food ad libitum. All the experiments performed on and the care given to the animals were developed in accordance with the standards of the University of Zaragoza and the international guide for the use of laboratory animals. A total of 12 animals were used: wild-type (n=5), SOD1G93A mice injected with pcDNA3.1 (control, n=5) and SOD1G93A mice treated with HcTeTx (n=5).

### 1.3 Naked DNA intramuscular injection and extraction of the spinal cord

At the age of 8 weeks the SOD1 G93A transgenic mice were injected intramuscularly with 300 µg of pCMV-HcTeTx in the cuadriceps muscle (two injections of 50 µg per muscle) and in the triceps muscle (one single injection of 50 µg per muscle). The control group of mice was injected with the same amounts of an empty plasmid.

110 days after the intramuscular plasmid injections, the spinal cords were extracted that were pre-frozen in liquid nitrogen and later stored at -70 °C. The tissues were frozen in liquid nitrogen and were ground to a powder thereafter in a cold mortar. Half of the sample was used to extract RNA and the other half was used for protein extraction.

### 1.4 Extraction of RNA from the spinal cord and cDNA synthesis

The total RNA from the spinal cords was extracted in accordance with the RNeasy® Lipid Tissue Mini Kit (Qiagen) Protocol. For cDNA synthesis, the SuperScriptTM First-Strand Synthesis System (Invitrogen) kit was used, beginning with 1 µg of RNA in a final volume of 20µL.

### 1.5 Real time PCR

PCR reactions in real time were carried out on a final volume of 10µL. with 1X of TaqMan® Universal PCR Master Mix, No AmpErase® UNG (Applied Biosystems), 1X of the unmarked initiator mix and TaqMan® MGB (Applied Biosystems) probes for each gene studied and 1µL per cDNA reaction diluted 10 times. For standardization 3 endogenous genes (18s rRNA, GAPDH and β-actin) were used. The references to the mixture of initiator and probes used to amplify each gene studied were the following: caspasa-3 (Mm00438023_m1), caspasa-1 (Mm00438023_m1), NCS-1 (Mm00490552_m1), Rrad (Mm00451053_m1), 18s rRNA (Hs99999901), GAPDH (4352932E) and β-actin (4352933E). All the PCR reactions were carried out in an ABI Prism 7000 Sequence Detection System thermal cycler (Applied Biosystems). The thermal cycle parameters were as follows: incubation at 94°C for 10 min and 40 cycles at 94°C for 15s and 60°C for 1s. The expression relative to caspase-3, caspase-1, NCS-1 and Rrad were standardized applying the geometric average of the three endogenous genes.

### 1.6 Extraction of the protein from the spinal cord and Western Blot analysis

The samples of the wild-type mice's spinal cords and the SOD1G93A mice treated with HcTeTx were homogenized in liquid nitrogen with the extraction buffer having the following composition: 150mM NaCl, 50mM Tris-HCl pH=7,5, 1% desoxicolate, 0,1% SDS, 1% Triton X-100, 1mM NaOVa, 1 mM PMSF, 10µg/mL leupeptin y aprotinin and 1 µg/mL pepstatin. It was centrifuged at 4 °C for 10 minutes at 3000 g. Once the protein concentration of the supernatant from each sample was quantified using the BCA (9643 Sigma) method, 25µg of the protein was loaded into a 10% acrylamide gel. PVDF membranes were used for the transfer process, which were blocked with a TTBS solution of 5% skim milk (20mM Tris base, 0.15M NaCl, pH=7.5, 0.1% Tween) for one hour. Later they were incubated with the primary antibody all night at 4°C (anti-p-Akt (sc-7985R, Santa Cruz)). GAPDH (glyceraldehydes 3 phosphate dehydrogenase) was used to standardize the medium obtained with Akt (anti-GAPDH (sc-25778, Santa Cruz)). After incubating the primary antibody, the membranes were washed with TTBS and they were incubated with the secondary antibody for 1 hour at room temperature. Finally, they were developed using chemical luminescence (Western Blotting Luminol Reagent, sc-2048 Santa Cruz). The films were scanned and analyzed using AlphaEase FC (Bonsai Technologies) software. The statistical analysis was carried out using the ANOVA test and the Student-Neuman-Keuls test.

### Results

In this study, results are presented from the application of HcTeTx in SOD1G93A ALS disease model mice, where a degeneration of motor neurons is present. The transcriptional study of the spinal cords of these mice in symptomatic stage is shown in Figure 7. Comparing the transcriptional regulation of the caspase-1, caspase-3, Bax and Bcl2 genes involved in apoptosis in the late onset symptomatic stage (110 days of age) in the spinal cord of wild-type mice and SOD1G93A mice. The results showed a significant induction of caspase-1 (P<0.05), caspase-3 (P<0.05) and Bcl2 (P<0.01) genes, but there was no significant difference in the profile of the Bax (P>0.05) gene in the SOD1 G93A control mice when they were compared with the wild-type (Figure 7). In the group of mice that received the HcTeTx treatment, the levels of caspase-1 and caspase-3 expression were maintained in the wild-type and significant differences were only found when compared with the untreated mice (P<0.05 and P<0.01, respectively). However, the expression of the Bax and Bcl2 genes was not affected by the hct treatment (P>0.05) in the spinal cords of these transgenic mice (Figure 7).

To evaluate the effects of HcTeTx on the mechanisms that revert the apoptosis that may induce cellular death in the spinal cords of the SOD1G93A mice, a protein study was also carried out. The data revealed that activation of the caspase-3 (P<0.05) gene decreased perceptibly in the mice treated with HcTeTx in comparison with the control group, reaching levels similar to the wild-type mice, while the levels of the pro-caspase-3 protein in the transgenic animals was not affected. In contrast with the results obtained from the expression analysis, in the Western-Blot test, it was observed that the amount of Bax and Bcl2 proteins were lower than in the mice treated with HcTeTx (Figure 8).

One HcTeTx method of action is the phosphorilation of Akt (Gil et al., 2003. Biochem J. 373:613-620), a kinase protein that is activated by various growth factors involved in blocking pathways mediated by phosphatidylinositol 3-kinase. The densiometric quantification indicated that the animals treated with HcTeTx had more than two time the levels of phosphorilated Akt in Ser473 when compared to the empty vector control (P<0.05), according to that determined by Western-Blot analysis using phosphor-specific antibodies (Figure 9). The equimolar load of proteins was confirmed by detection using anti-tubulin antibodies. The phosphorilation of ERK1/2 by HcTeTx has been disclosed previously in cultivated cortical neurons (Gil et al., 2003. Biochem J. 373:613-620). To confirm the involvement of HcTeTx in the MAP kinase pathway, Western-Blot analyses of the spinal cord extracts from the SOD1G93A treated and untreated mice at 110 days of age were performed. The result showed a growing activation of ERK1/2 in the control mice when they were compared with a group treated with HcTeTx (Figure 9), but the level of expression was similar to that of the wild-type mice.

### EXAMPLE 3

**Survival increase in SOD1G93A model mice for amyotrophic lateral sclerosis after administration of an intraperitoneal injection of a polypeptide that included the C-Terminal Domain of the heavy chain of the tetanus toxin (HcTeTx).**

### Materials and methods

### 1.1 Extraction of the Polypeptide that includes the C-Terminal Domain of the heavy chain of the tetanus toxin (HcTeTx)

The polypeptide used (called HcTeTx) corresponds to the C-Terminal Domain of the heavy chain of the tetanus toxin and includes the sequence of 451 amino acids (SEQ ID NO: 1) of the SEQ ID NO: 2, and has been obtained following the protocol described by Gil et al..(Gil etal., 2003. Biochem.J. 373,613-620).

### 1.2 Transgenic mice

Transgenic mice that overexpress human SOD1 with the G93A mutation (B6SJL-TgN[SOD1-G93A]1Gur) were obtained from the Jackson Laboratory (Bar Harbor, ME). Hemizygote mutants were used in all of the experiments (a mutant male mated to a non-transgenic female). The transgenic mice were identified by PCR amplification of the DNA extracted from the tail, as described in Gurney et al. (Gurney et al., 1994. Science, 264 (5166): 1772-5). The animals were preserved in a Mixed Research Unit at the University of Zaragoza. They were given water and food ad libitum. All the experiments performed on and the care given to the animals were developed in accordance with the standards of the University of Zaragoza and the international guide for the use of laboratory animals.

### 1.3 Intraperitoneal injection of the polypeptide in the animals_{.}

At the age of 12 weeks, the SOD1 G93A transgenic mice were injected intraperitoneally with 250 µl at a concentration of 0.5 µM of the polypeptide that includes the C-Terminal Domain of the tetanus toxin (HcTeTx). The injection was repeated weekly throughout their entire lives.

### 1.4 Measurement of survival in the animals.

The final point in the life of the mice was considered when the animal was placed in a supine position and was not able to turn themselves over.

### Results

### 1.1.- HcTeTx prolonged survival of the SOD1G93A transgenic mice

As can be seen in Figure 10, and in Table 2, maximum survival was detected in the mice from the group treated with HcTeTx, which reached an average of 135 days; 9 days longer than the control group.

**Table 2 Shows survival data, in the control group as well as in the group treated with HcTeTx, as well as the P Value.**

| | **Control** (n=3) | **HcTeTx** (n=3) | *P Value* |
|---|---|---|---|
| Mortality | 126 ± 4 | 135 ± 2 | 0,021 |

### EXAMPLE 4

### The administration of HcTeTx causes changes in the expression of genes related to calcium in the spinal cords of the SOD1G93A mice.

There is evidence of abnormal intracellular calcium homeostasis related to Amyotrophic Lateral Sclerosis (ALS). The Neuron protein NCS1 has been shown to regulate neurosecretion in a calcium dependent manner (McFerran et al., 1998. J. Biol. Chem. 273: 22768-22772) and also has been linked to the modulation of the calcium/calmodulin dependent enzymes involved in the neuronal signal transduction (Schaad et al., 1996. PNAS. 93: 9253-9258). The NCS1 expression in the spinal cords of the SOD1 G93A mice was tested 50 after treatment with HcTeTx. In the R-PCR experiments, the expression of the NCS1 gene was found to be repressed (P<0.05) in transgenic mice with late onset symptomatology with regard to the wild-type mice of the same age. Moreover, mice receiving the intramuscular HcTeTx treatment had higher levels of NCS1 (P<0.05), approaching those of the wild-type mice. With the same samples, the levels of messenger RNA were analyzed in the gen related to Ras and associated with the diabetes gene (Rrad). In this example the Rrad levels were increased almost two times in the spinal cord of the control transgenic mice, when they were compared with wild-type mice of a comparable age. However, in comparison with the controls, the treatment with HcTeTx in SOD1G93A mice perceptibly reduced the expression Rrad (P<0.05), reaching values similar to those obtained in the wild-type mice (Figure 11).

## Claims

1. Use of an isolated polynucleotide consisting of the encoding sequence of the carboxy-terminal domain of the heavy subunit of the tetanus toxin (HcTeTx), or a fragment of the encoding sequence, or a polynucleotide variant thereof, as a therapeutic agent in the manufacture of a medicament for the treatment of the symptoms of Amyotrophic Lateral Sclerosis (ALS), wherein
(a) the polypeptide encoded by the fragment or the polynucleotide variant maintains a therapeutic effect on ALS;
(b) the sequence of the polypeptide encoded by said polynucleotide variant is at least 85% identical to the sequence of SEQ ID NO:2 or SEQ ID NO:5;
(c) the polypeptide encoded by the isolated polynucleotide does not form part of a fusion protein; and
(d) the HcTeTx, HcTeTx fragment, or polypeptide variant encoded by the isolated polynucleotide is therapeutically effective by itself.

2. Use according to claim 1, wherein said encoding sequence of the carboxy-terminal domain of the heavy subunit of the tetanus toxin (HcTeTx) is SEQ ID NO: 1.

3. Use according to claim 1, wherein said encoding sequence is SEQ ID NO: 6.

4. Use according to claim 1, wherein said polynucleotide is to be administered as naked DNA.

5. Use according to claim 1, wherein said polynucleotide is to be administered orally, parenterally, intramuscularly, or nasally.

6. Use according to claim 1, wherein said polynucleotide is to be administered into a muscle.

7. Use according to claim 6, wherein said polynucleotide is to be expressed *in vivo* in said muscle.

8. Use according to claim 1, wherein said polynucleotide is inserted into an expression vector.

9. Use according to claim 8, wherein said vector is capable of *in vivo* expression.

10. Use according to claim 9, wherein said vector comprises a promoter capable of expressing the isolated polynucleotide contained in said vector.

11. Use according to claim 8, wherein said vector is the pcDNA3.1 expression vector.

12. Use according to claim 11, wherein said promoter is pCMV.

13. Use of an isolated polypeptide consisting of the carboxy-terminal domain of the heavy subunit of the tetanus toxin (HcTeTx), a HcTeTx fragment, or a polypeptide variant, as a therapeutic agent, for the manufacture of a medicament for the treatment of the symptoms of Amyotrophic Lateral Sclerosis (ALS), wherein:
(a) the HcTeTx fragment, or the polypeptide variant maintain a therapeutic effect on ALS;
(b) the sequence of the polypeptide variant is at least 85% identical to the sequence of SEQ ID NO:2 or SEQ ID NO:5;
(c) the HcTeTx, HcTeTx fragment, or polypeptide variant do not form part of a fusion protein; and,
(e) the HcTeTx, HcTeTx fragment, or polypeptide variant is therapeutically effective by itself.

14. Use according to claim 13, wherein said isolated polypeptide is the carboxy-terminal domain of the heavy subunit of the tetanus toxin (HcTeTx) (SEQ ID NO: 2).

15. Use according to claim 13, wherein said isolated polypeptide is SEQ ID NO: 5.

16. Use according to claim 13, wherein said polypeptide is to be administered orally, parenterally, intramuscularly, or nasally.

17. Use according to claim 13, wherein said polypeptide is to be administered intraperitoneally.

## Patentansprüche

1. Verwendung eines isolierten Polynukleotids, bestehend aus der kodierenden Sequenz der Carboxy-terminalen Domäne der schweren Untereinheit des Tetanus-Toxins (HcTeTx), oder aus einem Fragment der kodierenden Sequenz oder einer Polynukleotid-Variante davon, als einem therapeutischen Mittel in der Herstellung eines Medikaments für die Behandlung der Symptome von Amyothropher Lateralsklerose (ALS), wobei
(a) das Polypeptid, das durch das Fragment oder die Polynukleotid-Variante kodiert ist, eine therapeutische Wirkung auf ALS beibehält;
(b) die Sequenz des Polypeptids, das durch die Polynukleotid-Variante kodiert ist, zu wenigstens 85% identisch zu der Sequenz von SEQ ID NO: 2 oder SEQ ID NO: 5 ist;
(c) das Polypeptid, das durch das isolierte Polynukleotid kodiert ist, nicht Teil eines Fusionsproteins bildet; und
(d) das HcTeTx, HcTeTx-Fragment oder Polypeptid-Variante, das durch das isolierte Polynukleotid kodiert ist, selbst therapeutisch wirksam ist.

2. Verwendung gemäß Anspruch 1, wobei die kodierende Sequenz der Carboxy-terminalen Domäne der schweren Untereinheit des Tetanus-Toxins (HcTeTx) die SEQ ID NO: 1 ist.

3. Verwendung gemäß Anspruch 1, wobei die kodierende Sequenz die SEQ ID NO: 6 ist.

4. Verwendung gemäß Anspruch 1, wobei das Polynukleotid als nackte DNA zu verabreichen ist.

5. Verwendung gemäß Anspruch 1, wobei das Polynukleotid oral, parenteral, intramuskulär oder nasal zu verabreichen ist.

6. Verwendung gemäß Anspruch 1, wobei das Polynukleotid in einen Muskel zu verabreichen ist.

7. Verwendung gemäß Anspruch 6, wobei das Polynukleotid *in vivo* in dem Muskel zu exprimieren ist.

8. Verwendung gemäß Anspruch 1, wobei das Polynukleotid in einen Expressionsvektor insertiert wird.

9. Verwendung gemäß Anspruch 8, wobei der Vektor zur *in vivo*-Expression fähig ist.

10. Verwendung gemäß Anspruch 9, wobei der Vektor einen Promotor umfasst, der zum Exprimieren des in dem Vektor enthaltenen isolierten Polynukleotids fähig ist.

11. Verwendung gemäß Anspruch 8, wobei der Vektor der pcDNA3.1-Expressionsvektor ist.

12. Verwendung gemäß Anspruch 11, wobei der Promotor pCMV ist.

13. Verwendung eines isolierten Polypeptids, bestehend aus der Carboxy-terminalen Domäne der schweren Untereinheit des Tetanus-Toxins (HcTeTx), einem HcTeTx-Fragment oder einer Polypeptid-Variante, als einem therapeutischen Mittel für die Herstellung eines Medikaments für die Behandlung der Symptome von Amyothropher Lateralsklerose (ALS), wobei
(a) das HcTeTx-Fragment oder die Polypeptid-Variante eine therapeutische Wirkung auf ALS beibehält;
(b) die Sequenz der Polypeptid-Variante zu wenigstens 85% identisch zu der Sequenz von SEQ ID NO: 2 oder SEQ ID NO: 5 ist;
(c) das HcTeTx, HcTeTx-Fragment oder Polypeptid-Variante nicht Teil eines Fusionsproteins bildet; und
(d) das HcTeTx, HcTeTx-Fragment oder Polypeptid-Variante selbst therapeutisch wirksam ist.

14. Verwendung gemäß Anspruch 13, wobei das isolierte Polypeptid die Carboxyterminale Domäne der schweren Untereinheit des Tetanus-Toxins (HcTeTx) (SEQ ID NO: 2) ist.

15. Verwendung gemäß Anspruch 13, wobei das isolierte Polypeptid SEQ ID NO: 5 ist.

16. Verwendung gemäß Anspruch 13, wobei das Polypeptid oral, parenteral, intramuskulär oder nasal zu verabreichen ist.

17. Verwendung gemäß Anspruch 13, wobei das Polypeptid intraperitoneal zu verabreichen ist.

## Revendications

1. Utilisation d'un polynucléotide isolé consistant en la séquence codante du domaine carboxy-terminal de la sous-unité lourde de la toxine tétanique (HcTeTx) ou un fragment de la séquence codante ou une variante polynucléotidique de celle-ci, comme agent thérapeutique dans la production d'un médicament pour le traitement des symptômes de la sclérose latérale amyotrophique (SLA), dans laquelle
(a) le polypeptide codé par le fragment ou la variante polypeptidique conserve un effet thérapeutique sur la SLA ;
(b) la séquence du polypeptide codé par ladite variante polypeptidique est identique à au moins 85% à la séquence de SEQ ID N° : 2 ou de SEQ ID N° : 5 ;
(c) le polypeptide codé par le polynucléotide isolé ne fait pas partie d'une protéine de fusion ; et
(d) la HcTeTx, un fragment de HcTeTx ou une variante polypeptidique codée par le polynucléotide isolé est thérapeutiquement efficace en elle-même/lui-même.

2. Utilisation selon la revendication 1, dans laquelle ladite séquence codante du domaine carboxy-terminal de la sous-unité lourde de la toxine tétanique (HcTeTx) est SEQ ID N° : 1.

3. Utilisation selon la revendication 1, dans laquelle ladite séquence codante est SEQ ID N° : 6.

4. Utilisation selon la revendication 1, dans laquelle ledit polynucléotide doit être administré en tant qu'ADN nu.

5. Utilisation selon la revendication 1, dans laquelle ledit polynucléotide doit être administré par voie orale, parentérale, intramusculaire ou nasale.

6. Utilisation selon la revendication 1, dans laquelle ledit polynucléotide doit être administré dans un muscle.

7. Utilisation selon la revendication 6, dans laquelle ledit polynucléotide doit être exprimé *in vivo* dans ledit muscle.

8. Utilisation selon la revendication 1, dans laquelle ledit polynucléotide est inséré dans un vecteur d'expression.

9. Utilisation selon la revendication 8, dans laquelle ledit vecteur est capable d'être exprimé *in vivo*.

10. Utilisation selon la revendication 9, dans laquelle ledit vecteur comprend un promoteur capable d'exprimer le polynucléotide isolé contenu dans ledit vecteur.

11. Utilisation selon la revendication 8, dans laquelle ledit vecteur est le vecteur d'expression pcDNA3.1.

12. Utilisation selon la revendication 11, dans laquelle ledit promoteur est pCMV.

13. Utilisation d'un polypeptide isolé consistant en le domaine carboxy-terminal de la sous-unité lourde de la toxine tétanique (HcTeTx), un fragment de HcTeTx ou une variante polynucléotidique comme agent thérapeutique, pour la production d'un médicament pour le traitement des symptômes de la sclérose latérale amyotrophique (SLA), dans laquelle :
(a) le fragment de HcTeTx ou la variante polypeptidique conservent un effet thérapeutique sur la SLA ;
(b) la séquence de la variante polypeptidique est identique à au moins 85% à la séquence de SEQ ID N° : 2 ou de SEQ ID N° : 5 ;
(c) la HcTeTx, un fragment de HcTeTx ou la variante polypeptidique ne font pas partie d'une protéine de fusion ; et
(e) la HcTeTx, un fragment de HcTeTx ou une variante polypeptidique est thérapeutiquement efficace en elle-même/lui-même.

14. Utilisation selon la revendication 13, dans laquelle ledit polypeptide isolé est le domaine carboxy-terminal de la sous-unité lourde de la toxine tétanique (HcTeTx) (SEQ ID N° : 2).

15. Utilisation selon 13, dans laquelle ledit polypeptide isolé est SEQ ID N° : 5.

16. Utilisation selon la revendication 13, dans laquelle ledit polypeptide doit être administré par voie orale, parentérale, intramusculaire ou nasale.

17. Utilisation selon la revendication 13, dans laquelle ledit polypeptide doit être administré par voie intrapéritonéale.


### SEQUENCE LISTING

<110> UNIVERSIDAD DE ZARAGOZA UNIVERSIDAD DE AUTONOMA DE BARCELONA
<120> USE OF THE ENCODING SEQUENCE OF THE CARBOXY-TERMINAL DOMAIN OF
   THE HEAVY CHAIN OF THE TETANIC TOXIC AS A MEDICAMENT.
<130> ES1510.16
<160> 6
<170> PatentIn version 3.4
<210> 1
   <211> 1392
   <212> DNA
   <213> Clostridium tetani
<400> 1
   atggtttttt caacaccaat tccattttct tattctaaaa atctggattg ttgggttgat 60
   aatgaagaag atatagatgt tatattaaaa aagagtacaa ttttaaattt agatattaat 120
   aatgatatta tatcagatat atctgggttt aattcatctg taataacata tccagatgct 180
   caattggtgc ccggaataaa tggcaaagca atacatttag taaacaatga atcttctgaa 240
   gttatagtgc ataaagctat ggatattgaa tataatgata tgtttaataa ttttaccgtt 300
   agcttttggt tgagggttcc taaagtatct gctagtcatt tagaacaata tggcacaaat 360
   gagtattcaa taattagctc tatgaaaaaa catagtctat caataggatc tggttggagt 420
   gtatcactta aaggtaataa cttaatatgg actttaaaag attccgcggg agaagttaga 480
   caaataactt ttagggattt acctgataaa tttaatgctt atttagcaaa taaatgggtt 540
   tttataacta ttactaatga tagattatct tctgctaatt tgtatataaa tggagtactt 600
   atgggaagtg cagaaattac tggtttagga gctattagag aggataataa tataacatta 660
   aaactagata gatgtaataa taataatcaa tacgtttcta ttgataaatt taggatattt 720
   tgcaaagcat taaatccaaa agagattgaa aaattataca caagttattt atctataacc 780
   tttttaagag acttctgggg aaacccttta cgatatgata cagaatatta tttaatacca 840
   gtagcttcta gttctaaaga tgttcaattg aaaaatataa cagattatat gtatttgaca 900
   aatgcgccat cgtatactaa cggaaaattg aatatatatt atagaaggtt atataatgga 960
   ctaaaattta ttataaaaag atatacacct aataatgaaa tagattcttt tgttaaatca 1020
   ggtgatttta ttaaattata tgtatcatat aacaataatg agcacattgt aggttatccg 1080
   aaagatggaa atgcctttaa taatcttgat agaattctaa gagtaggtta taatgcccca 1140
   ggtatccctc tttataaaaa aatggaagca gtaaaattgc gtgatttaaa aacctattct 1200
   gtacaactta aattatatga tgataaaaat gcatctttag gactagtagg tacccataat 1260
   ggtcaaatag gcaacgatcc aaatagggat atattaattg caagcaactg gtactttaat 1320
   catttaaaag ataaaatttt aggatgtgat tggtactttg tacctacaga tgagggatgg 1380
   acaaatgatt aa 1392
<210> 2
   <211> 462
   <212> PRT
   <213> clostridium tetani
<400> 2
   Val Phe Ser Thr Pro Ile Pro Phe Ser Tyr Ser Lys Asn Leu Asp Cys
   1               5                   10                  15
   Trp Val Asp Asn Glu Glu Asp Ile Asp Val Ile Leu Lys Lys Ser Thr
               20                  25                  30
   Ile Leu Asn Leu Asp Ile Asn Asn Asp Ile Ile Ser Asp Ile Ser Gly
           35                  40                  45
   Phe Asn Ser Ser Val Ile Thr Tyr Pro Asp Ala Gln Leu Val Pro Gly
       50                  55                  60
   Ile Asn Gly Lys Ala Ile His Leu Val Asn Asn Glu Ser Ser Glu Val
   65                  70                  75                  80
   Ile Val His Lys Ala Met Asp Ile Glu Tyr Asn Asp Met Phe Asn Asn
                   85                  90                  95
   Phe Thr Val Ser Phe Trp Leu Arg Val Pro Lys Val Ser Ala Ser His
               100                 105                 110
   Leu Glu Gln Tyr Gly Thr Asn Glu Tyr Ser Ile Ile Ser Ser Met Lys
           115                 120                 125
   Lys His Ser Leu Ser Ile Gly Ser Gly Trp Ser Val Ser Leu Lys Gly
       130                 135                 140
   Asn Asn Leu Ile Trp Thr Leu Lys Asp Ser Ala Gly Glu Val Arg Gln
   145                 150                 155                 160
   Ile Thr Phe Arg Asp Leu Pro Asp Lys Phe Asn Ala Tyr Leu Ala Asn
                   165                 170                 175
   Lys Trp Val Phe Ile Thr Ile Thr Asn Asp Arg Leu Ser Ser Ala Asn
               180                 185                 190
   Leu Tyr Ile Asn Gly Val Leu Met Gly Ser Ala Glu Ile Thr Gly Leu
           195                 200                 205
   Gly Ala Ile Arg Glu Asp Asn Asn Ile Thr Leu Lys Leu Asp Arg Cys
       210                 215                 220
   Asn Asn Asn Asn Gln Tyr Val Ser Ile Asp Lys Phe Arg Ile Phe Cys
   225                 230                 235                 240
   Lys Ala Leu Asn Pro Lys Glu Ile Glu Lys Leu Tyr Thr Ser Tyr Leu
                   245                 250                 255
   Ser Ile Thr Phe Leu Arg Asp Phe Trp Gly Asn Pro Leu Arg Tyr Asp
               260                 265                 270
   Thr Glu Tyr Tyr Leu Ile Pro Val Ala Ser Ser Ser Lys Asp Val Gln
           275                 280                 285
   Leu Lys Asn Ile Thr Asp Tyr Met Tyr Leu Thr Asn Ala Pro Ser Tyr
       290                 295                 300
   Thr Asn Gly Lys Leu Asn Ile Tyr Tyr Arg Arg Leu Tyr Asn Gly Leu
   305                 310                 315                 320
   Lys Phe Ile Ile Lys Arg Tyr Thr Pro Asn Asn Glu Ile Asp Ser Phe
                   325                 330                 335
   Val Lys Ser Gly Asp Phe Ile Lys Leu Tyr Val Ser Tyr Asn Asn Asn
               340                 345                 350
   Glu His Ile Val Gly Tyr Pro Lys Asp Gly Asn Ala Phe Asn Asn Leu
           355                 360                 365
   Asp Arg Ile Leu Arg Val Gly Tyr Asn Ala Pro Gly Ile Pro Leu Tyr
       370                 375                 380
   Lys Lys Met Glu Ala Val Lys Leu Arg Asp Leu Lys Thr Tyr Ser Val
   385                 390                 395                 400
   Gln Leu Lys Leu Tyr Asp Asp Lys Asn Ala Ser Leu Gly Leu Val Gly
                   405                 410                 415
   Thr His Asn Gly Gln Ile Gly Asn Asp Pro Asn Arg Asp Ile Leu Ile
               420                 425                 430
   Ala Ser Asn Trp Tyr Phe Asn His Leu Lys Asp Lys Ile Leu Gly Cys
           435                 440                 445
   Asp Trp Tyr Phe Val Pro Thr Asp Glu Gly Trp Thr Asn Asp
       450                 455                 460
<210> 3
   <211> 21
   <212> DNA
   <213> Artificial sequence (primer)
<400> 3
   agattccgcg ggagaagtta g 21
<210> 4
   <211> 21
   <212> DNA
   <213> Artificial sequence (primer)
<400> 4
   tcgtaaaggg tttccccaga a 21
<210> 5
   <211> 451
   <212> PRT
   <213> clostridium tetani
<220>
   <221> MISC_FEATURE
   <222> (1)..(451)
   <223> Fragment of SEQ ID NO:2
<400> 5
   Lys Asn Leu Asp Cys Trp Val Asp Asn Glu Glu Asp Ile Asp Val Ile
   1               5                   10                  15
   Leu Lys Lys Ser Thr Ile Leu Asn Leu Asp Ile Asn Asn Asp Ile Ile
               20                  25                  30
   Ser Asp Ile Ser Gly Phe Asn Ser Ser Val Ile Thr Tyr Pro Asp Ala
           35                  40                  45
   Gln Leu Val Pro Gly Ile Asn Gly Lys Ala Ile His Leu Val Asn Asn
       50                  55                  60
   Glu Ser Ser Glu Val Ile Val His Lys Ala Met Asp Ile Glu Tyr Asn
   65                  70                  75                  80
   Asp Met Phe Asn Asn Phe Thr Val Ser Phe Trp Leu Arg Val Pro Lys
                   85                  90                  95
   Val Ser Ala Ser His Leu Glu Gln Tyr Gly Thr Asn Glu Tyr Ser Ile
               100                 105                 110
   Ile Ser Ser Met Lys Lys His Ser Leu Ser Ile Gly Ser Gly Trp Ser
           115                 120                 125
   Val Ser Leu Lys Gly Asn Asn Leu Ile Trp Thr Leu Lys Asp Ser Ala
       130                 135                 140
   Gly Glu Val Arg Gln Ile Thr Phe Arg Asp Leu Pro Asp Lys Phe Asn
   145                 150                 155                 160
   Ala Tyr Leu Ala Asn Lys Trp Val Phe Ile Thr Ile Thr Asn Asp Arg
                   165                 170                 175
   Leu Ser Ser Ala Asn Leu Tyr Ile Asn Gly Val Leu Met Gly Ser Ala
               180                 185                 190
   Glu Ile Thr Gly Leu Gly Ala Ile Arg Glu Asp Asn Asn Ile Thr Leu
           195                 200                 205
   Lys Leu Asp Arg Cys Asn Asn Asn Asn Gln Tyr Val Ser Ile Asp Lys
   210 215 220
   Phe Arg Ile Phe Cys Lys Ala Leu Asn Pro Lys Glu Ile Glu Lys Leu
   225 230 235 240
   Tyr Thr Ser Tyr Leu Ser Ile Thr Phe Leu Arg Asp Phe Trp Gly Asn
   245 250 255
   Pro Leu Arg Tyr Asp Thr Glu Tyr Tyr Leu Ile Pro Val Ala Ser Ser
   260 265 270
   Ser Lys Asp Val Gln Leu Lys Asn Ile Thr Asp Tyr Met Tyr Leu Thr
   275 280 285
   Asn Ala Pro Ser Tyr Thr Asn Gly Lys Leu Asn Ile Tyr Tyr Arg Arg
   290 295 300
   Leu Tyr Asn Gly Leu Lys Phe Ile Ile Lys Arg Tyr Thr Pro Asn Asn
   305 310 315 320
   Glu Ile Asp Ser Phe Val Lys Ser Gly Asp Phe Ile Lys Leu Tyr Val
   325 330 335
   Ser Tyr Asn Asn Asn Glu His Ile Val Gly Tyr Pro Lys Asp Gly Asn
   340 345 350
   Ala Phe Asn Asn Leu Asp Arg Ile Leu Arg Val Gly Tyr Asn Ala Pro
   355 360 365
   Gly Ile Pro Leu Tyr Lys Lys Met Glu Ala Val Lys Leu Arg Asp Leu
   370 375 380
   Lys Thr Tyr Ser Val Gln Leu Lys Leu Tyr Asp Asp Lys Asn Ala Ser
   385 390 395 400
   Leu Gly Leu Val Gly Thr His Asn Gly Gln Ile Gly Asn Asp Pro Asn
   405 410 415
   Arg Asp Ile Leu Ile Ala Ser Asn Trp Tyr Phe Asn His Leu Lys Asp
   420 425 430
   Lys Ile Leu Gly Cys Asp Trp Tyr Phe Val Pro Thr Asp Glu Gly Trp
   435 440 445
   Thr Asn Asp
   450
<210> 6
   <211> 1359
   <212> DNA
   <213> clostridium tetani
<400> 6
   atgaaaaatc tggattgttg ggttgataat gaagaagata tagatgttat attaaaaaag   60
   agtacaattt taaatttaga tattaataat gatattatat cagatatatc tgggtttaat  120
   tcatctgtaa taacatatcc agatgctcaa ttggtgcccg gaataaatgg caaagcaata  180
   catttagtaa acaatgaatc ttctgaagtt atagtgcata aagctatgga tattgaatat  240
   aatgatatgt ttaataattt taccgttagc ttttggttga gggttcctaa agtatctgct  300
   agtcatttag aacaatatgg cacaaatgag tattcaataa ttagctctat gaaaaaacat  360
   agtctatcaa taggatctgg ttggagtgta tcacttaaag gtaataactt aatatggact  420
   ttaaaagatt ccgcgggaga agttagacaa ataactttta gggatttacc tgataaattt  480
   aatgcttatt tagcaaataa atgggttttt ataactatta ctaatgatag attatcttct  540
   gctaatttgt atataaatgg agtacttatg ggaagtgcag aaattactgg tttaggagct  600
   attagagagg ataataatat aacattaaaa ctagatagat gtaataataa taatcaatac  660
   gtttctattg ataaatttag gatattttgc aaagcattaa atccaaaaga gattgaaaaa  720
   ttatacacaa gttatttatc tataaccttt ttaagagact tctggggaaa ccctttacga  780
   tatgatacag aatattattt aataccagta gcttctagtt ctaaagatgt tcaattgaaa  840
   aatataacag attatatgta tttgacaaat gcgccatcgt atactaacgg aaaattgaat  900
   atatattata gaaggttata taatggacta aaatttatta taaaaagata tacacctaat  960
   aatgaaatag attcttttgt taaatcaggt gattttatta aattatatgt atcatataac 1020
   aataatgagc acattgtagg ttatccgaaa gatggaaatg cctttaataa tcttgataga 1080
   attctaagag taggttataa tgccccaggt atccctcttt ataaaaaaat ggaagcagta 1140
   aaattgcgtg atttaaaaac ctattctgta caacttaaat tatatgatga taaaaatgca 1200
   tctttaggac tagtaggtac ccataatggt caaataggca acgatccaaa tagggatata 1260
   ttaattgcaa gcaactggta ctttaatcat ttaaaagata aaattttagg atgtgattgg 1320
   tactttgtac ctacagatga gggatggaca aatgattaa                        1359